# EUROPÄISCHE PATENTSCHRIFT

(11) **EP 3 360 583 B1**
(45) Veröffentlichungstag und Bekanntmachung des Hinweises auf die Patenterteilung: **25.03.2020**
(21) Anmeldenummer: 18153737.4
(22) Anmeldetag: 26.01.2018
(51) Int. Cl.: A61L 2/10

(54) **DESINFEKTIONS- UND TRANSPORTANORDNUNG**
DISINFECTING AND TRANSPORT ASSEMBLY
DISPOSITIF DE DÉSINFECTION ET DE TRANSPORT

(30) Priorität: 27.01.2017 DE 102017101624
(43) Veröffentlichungstag der Anmeldung: 15.08.2018
(73) Patentinhaber: Frontmatec - Hygiene GmbH, 59269 Beckum (DE)
(72) Erfinder: Quas genannt Möllmann, Martin, 59269 Beckum (DE)
(74) Vertreter: Andrejewski - Honke Patent- und Rechtsanwälte Partnerschaft mbB

(56) Entgegenhaltungen:
- JP-A- 2006 214 768
- RU-C1- 2 022 222
- US-A- 5 597 597
- US-A1- 2015 251 409

## Beschreibung

Die vorliegende Erfindung betrifft eine Desinfektions- und Transportanordnung für den Transport und Desinfektion von Produkten mit zumindest einer Transportrolle und einer in die Transportrolle eingesetzten ersten UV-Lichtquelle.

In der Praxis besteht ein großes Bedürfnis, verschiedene Produkte, einschließlich Verpackungen, zu desinfizieren, worunter im Rahmen der Erfindung insbesondere auch die Abtötung von Keimen und Mikroorganismen verstanden wird.

In diesem Zusammenhang hat sich der Einsatz von UV-Licht bewährt. Aufgrund der im Vergleich zu sichtbarem Licht hohen Photonenenergie ist UV-Licht in der Lage, Elektronen aus Atomen oder Molekülen zu lösen, sowie Molekülverbindungen zu zerstören. Durch eine solche Modifikation auf molekularer Basis werden Mikroorganismen wie Bakterien und Pilze inaktiviert. Selbst Viren können durch eine Schädigung der entsprechenden Nukleinsäuren unschädlich gemacht werden.

Für entsprechende Anwendungen wird häufig Strahlung im fernen UV-Bereich eingesetzt, der auch als UV-C-Bereich bezeichnet wird, Der Wellenlängenbereich des fernen UV liegt etwa bei 200 nm bis 280 nm, was einer Photonenenergie von 4,43 bis 6,20 eV entspricht. Handelsübliche UV-Lichtquellen sind insbesondere im Bereich einer bevorzugten Wellenlänge von 254 nm erhältlich.

Entsprechend wird UV-Licht für die Desinfektion und insbesondere Keimfreimachung von Wasser, Luft und unterschiedlichsten Produkten eingesetzt.

Die Bestrahlung mittels UV-Licht kann beispielsweise auch bei der Herstellung und Verarbeitung von Produkten wie Lebensmitteln integriert werden.

Bei solchen Produkten ist gerade eine Desinfizierung von Oberflächen besonders wichtig, weil diese einer großen Gefahr einer Kontaminierung ausgesetzt sind.

Bei der Integration in einen Produktionsprozess werden geringe Durchlaufzeiten sowie eine zuverlässige Desinfektion angestrebt, wobei auch bei der Handhabung der entsprechenden Produkte Abschattungseffekte zu vermeiden sind. Bei einer unvollständigen Bestrahlung mittels UV-Licht bleiben an den nicht bestrahlten Stellen aktive Mikroorganismen und Viren zurück. Es besteht sogar die Gefahr einer Re-Kontamination bereits desinfizierter Flächen.

Wenn die zu desinfizierenden Produkte beispielsweise in einem Durchlaufbetrieb in einem Desinfektionstunnel behandelt werden, ist der Einsatz von Förderbändern, Gitterförderern oder dergleichen schwierig, weil dann an den Auflageflächen die Gefahr einer unzureichenden Desinfektion besteht.

Vor diesem Hintergrund ist aus EP 2 705 858 A1 eine Desinfektions- und Transportanordnung nach Art eines Desinfektionstunnels bekannt, bei dem die Produkte innerhalb des Desinfektionstunnels auf einem Rollenförderabschnitt transportiert werden, wobei röhrenförmige UV-Lichtquellen einerseits oberhalb des Rollenförderabschnittes als auch andererseits in den Zwischenräumen der einzelnen Transportrollen angeordnet sind. Entsprechende Ausgestaltungen sind auch in US 4,877,964 und US 5,958,336 beschrieben.

Ein ähnlicher Ansatz unter Einbindung eines Förderbandes ist aus WO 2004/064874 A1 bekannt, wobei das Förderband an oberen Umlenkrollen die zu desinfizierenden Produkte trägt und sodann um untere Umlenkrollen um die röhrenförmigen UV-Lichtquellen herumgeführt ist.

Bei diesen aus dem Stand der Technik bekannten Ausgestaltungen ergibt sich der Nachteil, dass zwischen den aufeinanderfolgenden Transportrollen ein relativ großer Zwischenraum bereitgehalten werden muss, so dass nur relativ große Produkte behandelt werden können, die den Zwischenraum zwischen zwei aufeinanderfolgenden Transportrollen überbrücken können.

Eine gattungsgemäße Desinfektions- und Transportanordnung mit den eingangs beschriebenen Merkmalen ist aus WO 2013/173703 A1 bekannt. Röhrenförmige UV-Lichtquellen sind dabei in Förderrollen aus Quarz-Glas eingesetzt, welche für UV-Licht transparent sind. Die Bereitstellung entsprechend großer und tragfähiger Förderrollen aus Quarz-Glas ist mit relativ hohen Kosten verbunden, wobei auch eine mechanische Beschädigung des Quarz-Glases zu vermeiden ist.

Aus US 2015/0251409 A1 ist eine Druckeinrichtung bekannt, bei der eine UV-Lichtquelle in eine für das UV-Licht durchlässige Transportrolle für ein Förderband eingesetzt ist. Das ebenfalls für UV-Licht durchlässige Förderband ist um die Transportrolle mit der eingesetzten UV-Lichtquelle sowie um eine Antriebsrolle herumgeführt. Durch die Transportrolle und das Förderband hindurch kann ein zu bedruckendes Medium bei seinem Transport mit UV-Licht bestrahlt werden.

Vor diesem Hintergrund liegt der vorliegenden Erfindung die Aufgabe zugrunde, eine Desinfektions- und Transportanordnung insbesondere nach Art eines Desinfektionstunnels anzugeben, die sich durch eine besonders einfache und zuverlässige Handhabung auszeichnet, wobei auch ein Einsatz für Produkte mit unterschiedlichen Formen und Abmessungen ermöglicht werden soll.

Gegenstand der Erfindung und Lösung der Aufgabe ist eine Desinfektions- und Transportanordnung für den Transport und die Desinfektion von Produkten gemäß Patentanspruch 1.

Erfindungsgemäß ist vorgesehen, dass ein Mantel der Transportrolle aus einem für das von der ersten UV-Lichtquelle erzeugte UV-Licht undurchlässigen Material gebildet ist und Öffnungen für einen Durchtritt des UV-Lichtes aufweist.

Im Rahmen der Erfindung kann der Mantel aus einem Material gebildet sein, welches eine hohe Tragfähigkeit aufweist und auch ansonsten für die Bildung von Transportrollen bzw. die Mantelfläche von Transportrollen eingesetzt wird. Neben widerstandfähigen Kunststoffen kommt als besonders bevorzugtes Material für den Mantel Metall, insbesondere Edelstahl in Betracht. Während bei Kunststoffen bei einer dauerhaften UV-Bestrahlung die Gefahr einer Versprödung oder andersartigen Materialbeeinträchtigung besteht, ist Metall ohne eine Beeinträchtigung für einen langfristigen Einsatz geeignet. Edelstahl zeichnet sich darüber hinaus auch durch eine hohe Widerstandfähigkeit gegen andere Einflüsse wie beispielsweise Korrosion aus.

Bei der zylinderförmigen Fläche des Mantels bilden die Öffnungen eine Art Perforation. Insbesondere kann der Mantel Öffnungen in Form von Längsschlitzen und zwischen den Längsschlitzen verlaufende Stege aufweisen.

Aus Stabilitätsgründen ist es von Vorteil, wenn die Längsschlitze auch in Längsrichtung des zylinderförmigen Mantels verlaufen. Entlang der Länge des Mantels können mehrere Längsschlitze hintereinander vorgesehen sein, welche dann mit in Umfangsrichtung verlaufenden Stegen eine Reihe ergeben. Um den Umfang sind dann mehrere solcher Reihen angeordnet, wobei die Längsschlitze bzw. in Umfangrichtung verlaufenden Stege von benachbarten Reihen zweckmäßigerweise gegeneinander versetzt sind. Beispielsweise können die in Umfangrichtung verlaufenden Stege mittig in Bezug auf die in Umfangrichtung beidseitig anschließenden Längsschlitze angeordnet sein.

Die Form und der Flächenanteil der Öffnungen sowie der dazwischen verbleibenden Materialabschnitte ist so auszuwählen, dass einerseits möglichst viel UV-Licht hindurchtreten kann und andererseits eine ausreichende Stabilität gewährleistet wird. Vor diesem Hintergrund beträgt der Flächenanteil der Öffnungen an der Gesamtfläche des Mantels beispielsweise zwischen 40 % und 90 % vorzugsweise zwischen 50 % und 80 %.

Im Rahmen der Erfindung kommen unterschiedliche Arten von UV-Lichtquellen in Betracht. Beispielsweise können zylinderförmig ausgestaltete erste Lichtquellen in Form von UV-Röhren eingesetzt werden. Alternativ sind auch Halbleiterdioden als UV-Lichtquellen bekannt, wobei auch dann gemäß der Zylinderform des Mantels die UV-Lichtquelle durch eine Vielzahl von linienförmig angeordneten UV-Dioden gebildet sein kann. Die vorzugsweise zylinderförmige bzw. linienförmig ausgestaltete erste Lichtquelle ist zweckmäßigerweise ortsfest, also nicht drehbar angeordnet, wobei der zugeordnete Mantel um die Lichtquelle drehbar gelagert ist. Beispielsweise kann der Mantel über Wälz- oder Gleitlager um die erste Lichtquelle herum drehbar sein.

Um die zumindest eine Transportrolle drehbar antreiben zu können, kann der Mantel direkt oder über ein Zwischenstück an ein Antriebsrad angeschlossen sein. Beispielsweise kann ein Antriebsrad in Form eines Zahnrades von einer Kette oder einem Riemen angetrieben werden, wobei - wie nachfolgend weiter erläutert - auch eine Vielzahl von aufeinanderfolgenden Transportrollen in der beschriebenen Weise gemeinsam antreibbar ist.

Die Größe der Transportrollen und insbesondere des Mantels ist so auszuwählen, dass einerseits die Anordnung der ersten Lichtquelle möglich ist und andererseits ein möglichst ebener, gleichmäßiger Transport erfolgen kann. Vor diesem Hintergrund weist der Mantel besonders bevorzugt einen Außendurchmesser zwischen 30 mm und 80 mm, beispielsweise etwa 50 mm auf.

Die Desinfektions- und Transportanordnung weist vorzugsweise eine Vielzahl von Transportrollen auf, welche zumindest teilweise jeweils eine erste UV-Lichtquelle aufnehmen und gemeinsam einen Rollenförderabschnitt bilden. Je nach Bedarf können sämtliche Transportrollen des Rollenförderabschnittes mit einer ersten UV-Lichtquelle ausgerüstet sein, wobei darüber hinaus auch eine Kombination mit herkömmlichen Transportrollen ohne eine eingesetzte UV-Lichtquelle in Betracht kommt.

Zumindest die Transportrollen, welche eine erste UV-Lichtquelle aufnehmen, können in einem Gehäuse angeordnet sein, welches einen Desinfektionstunnel bildet. Ein solcher Desinfektionstunnel ist lediglich an seinen Enden geöffnet, um einen Eingang und einen Ausgang zu bilden. Das Gehäuse stellt dabei sicher, dass Personen und Gegenstände außerhalb des Desinfektionstunnels vor UV-Licht geschützt sind. Um hohen hygienischen Anforderungen gerecht zu werden und eine leichte Reinigung zu ermöglichen, kann das Gehäuse aus Edelstahl gebildet sein.

Das Gehäuse umfasst vorzugsweise eine Haube, welche zu Wartungs- und Reinigungszwecken abgenommen bzw. aufgeklappt werden kann. Nach einem Öffnen der Haube sind dann die Transportrollen zugänglich und können beispielsweise gereinigt werden, wobei auch eine Wartung, insbesondere ein Austausch fehlerhafter UV-Lichtquellen möglich ist.

Um die zu desinfizierenden Produkte vollständig bestrahlen zu können, sind im Rahmen der Erfindung zweckmäßigerweise weitere UV-Lichtquellen vorgesehen. Insbesondere können oberhalb und gegebenenfalls auch seitlich der Transportrollen zweite UV-Lichtquellen angeordnet sein, wobei dann eine vollständige Bestrahlung möglich ist. Auch die zweiten UV-Lichtquellen können bevorzugt als UV-Röhren ausgestaltet sein.

An dem Eingang und dem Ausgang des Desinfektionstunnels können für einen durchlaufenden Betrieb auch Vorhänge vorgesehen sein, welche den Austritt von UV-Licht vermeiden. Darüber hinaus kommen auch schleusenartige Verschlussmittel in Betracht, welche sich für den Durchlass einzelner Produkte öffnen und nachfolgend wieder schließen.

Im Rahmen der Erfindung können bei einer Ausgestaltung als Desinfektionstunnel mehrere Transportrollen innerhalb des Gehäuses einen Rollenförderabschnitt bilden, wobei dann an dem Eingang sowie dem Ausgang des Desinfektionstunnels weitere Förderabschnitte anschließen. Auch diese Förderabschnitte können als Rollenförderabschnitte ausgebildet sein, so dass sämtliche Rollenförderabschnitte einen kontinuierlichen Rollenförderer bilden. Rollenförderer zeichnen sich auch dadurch aus, dass sie in Produktionspausen bzw. nach einem Betriebsende gut vollständig gereinigt werden können. Außerhalb des Desinfektionstunnels ist jedoch auch ein Transport mittels Förderbändern, Rutschen oder anderen Einrichtungen möglich. Bei Förderbändern ergibt sich der Vorteil, dass diese bei einem Teil ihrer umlaufenden Bewegung von dem UV-Licht bestrahlt und somit im Sinne der Erfindung bei jedem Umlauf desinfiziert werden. Da die Bestrahlung andererseits nur über einen Teil, insbesondere einen kleinen Teil des gesamten Umlaufweges erfolgt, bleibt auch die Materialbelastung durch die UV-Strahlung relativ gering.

Die Erfindung wird nachfolgend anhand einer lediglich ein Ausführungsbeispiel darstellenden Zeichnung erläutert. Es zeigen:
- Fig. 1: Eine Desinfektions- und Transportanordnung für den Transport und die Desinfektion von Produkten,
- Fig. 2: eine Detailansicht der Desinfektions- und Transportanordnung gemäß Fig. 1 mit einem geöffneten Gehäuse,
- Fig. 3A: eine Transportrolle der Desinfektions- und Transportanordnung,
- Fig. 3B: ein Schnitt durch die Transportrolle gemäß der Fig. 3A,
- Fig. 4: eine Explosionsdarstellung der Transportrolle gemäß der Fig. 3A.

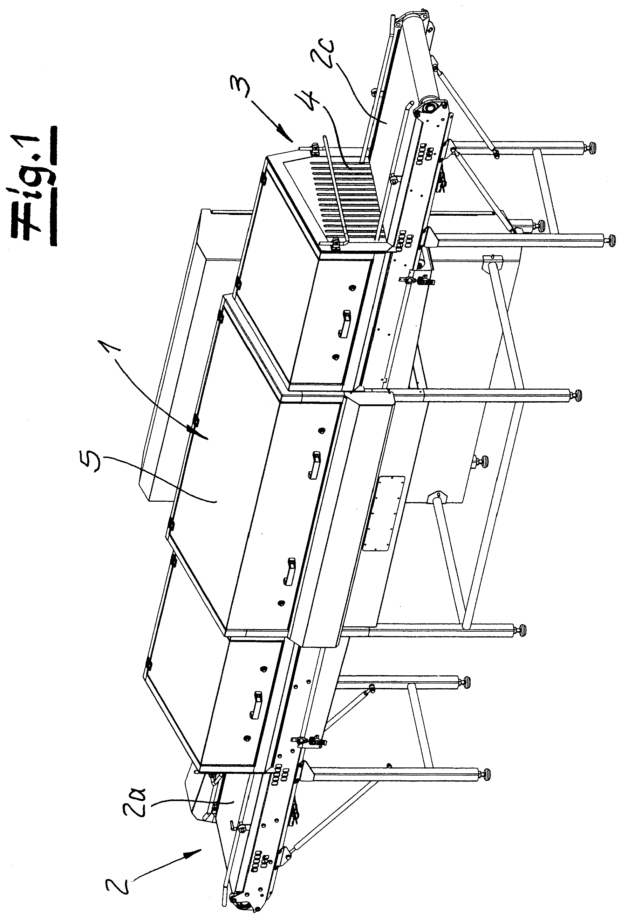

Die Fig. 1 zeigt eine Desinfektions- und Transportanordnung für den Transport und die Desinfektion von Produkten. Bei den Produkten kann es sich beispielsweise um vorbereitete Verpackungen sowie verpackte oder unverpackte Lebensmittel handeln, wobei mittels UV-Licht eine Desinfektion der Oberfläche erfolgen soll. Unter einer Desinfektion wird im Rahmen der Erfindung insbesondere eine zumindest teilweise Entfernung bzw. Deaktivierung von Mikroorganismen und Viren verstanden.

Gemäß der Fig. 1 weist die Desinfektions- und Transportanordnung nach Art eines Desinfektionstunnels ein Gehäuse 1 auf, wobei die nicht dargestellten Produkte mit einem Förderer 2 durch das Gehäuse 1 geführt werden können. Entlang des Förderers 2 weist der von dem Gehäuse 1 gebildete Desinfektionstunnel einen Eingang sowie einen Ausgang 3 auf, die jeweils durch einen Vorhang 4 verdeckt sind. Ein erster Förderabschnitt, im dargestellten Beispiel beim Bandförderabschnitt 2a, ist vor dem Eingang vorgesehen. An dem Eingang schließt dann ein zweiter Förderabschnitt innerhalb des Gehäuses 1 an, der als Rollenförderabschnitt 2b ausgestaltet ist, wobei dann an dem Ausgang 3 ein Bandförderabschnitt 2c als dritter Förderabschnitt vorhanden ist.

In der Fig. 1 ist ersichtlich, dass das Gehäuse 1 zumindest eine Haube 5 aufweist.

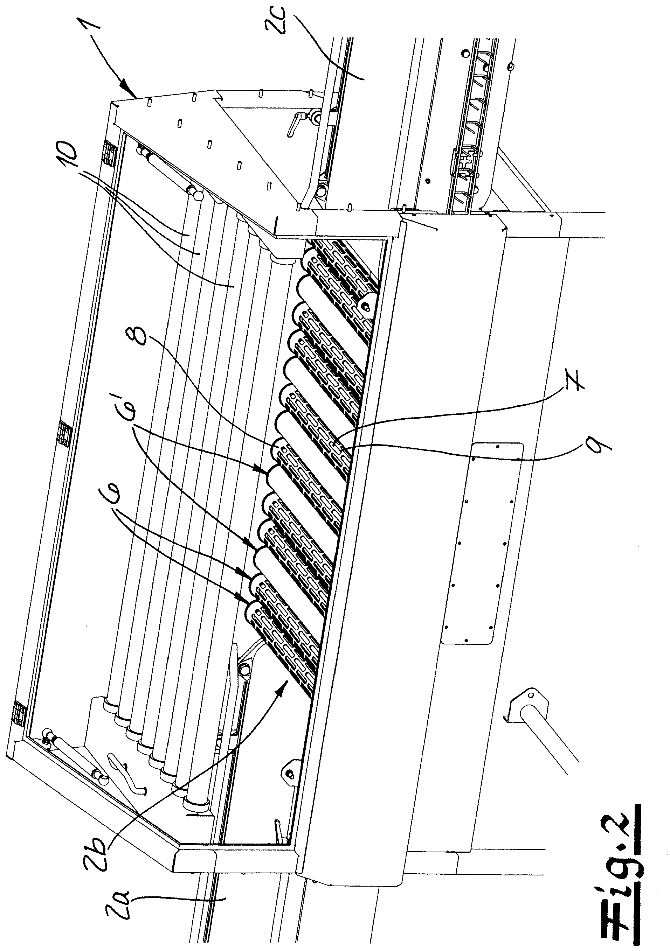

Die Fig. 2 zeigt eine Detailansicht der Desinfektions- und Transportanordnung mit einer entfernten Haube 5.

Es ist zu erkennen, dass der Rollenförderabschnitt 2b innerhalb des Gehäuses 1 in dem Ausführungsbeispiel unterschiedliche Transportrollen 6, 6' aufweist. Bei einer ersten Gruppe von Transportrollen 6 ist eine erste UV-Lichtquelle 7 eingesetzt, mit der die auf dem entsprechenden Rollenförderabschnitt 2b transportierten Produkte von unten bestrahlt werden können. Die entsprechenden Transportrollen 6 weisen dazu einen Mantel 8 aus einem für das UV-Licht undurchlässigen Material auf, wobei jedoch Öffnungen 9 in dem Mantel 8 einen Durchtritt des UV-Lichtes ermöglichen.

Als Material für den Mantel 8 ist bei den entsprechenden Transportrollen 6 Metall, insbesondere Edelstahl geeignet. Es ergibt sich der Vorteil, dass sämtliche Transportrollen 6, 6' in enger Folge angeordnet werden können und eine hohe Widerstandsfähigkeit und Tragkraft aufweisen. Insbesondere können durch die enge Anordnung sämtlicher Transportrollen 6, 6' auch vergleichsweise kleine Produkte durch die erfindungsgemäße Desinfektions- und Transportanordnung hindurchgeführt werden.

Oberhalb der Transportrollen 6, 6' befinden sich zweite UV-Lichtquellen 10, welche in dem dargestellten Ausführungsbeispiel als UV-Röhren ausgestaltet sind und entlang einer Längsrichtung des Gehäuses 1 quer zu den Transportrollen 6, 6' verlaufen. Durch die Anordnung der ersten UV-Lichtquellen 7 in den entsprechenden Transportrollen 6 sowie der zweiten UV-Lichtquellen 10 oberhalb der Transportrollen 6, 6' kann eine vollflächige Bestrahlung der zu desinfizierenden Produkte erfolgen.

Sämtliche Transportrollen 6, 6' sind gemeinsam angetrieben, um eine gleichmäßige Bewegung der zu desinfizierenden Produkte zu erreichen.

Die Transportrollen 6', welche nicht mit einer ersten UV-Lichtquelle 7 ausgerüstet sind, weisen eine übliche Bauform auf und müssen deshalb nicht weiter erläutert werden.

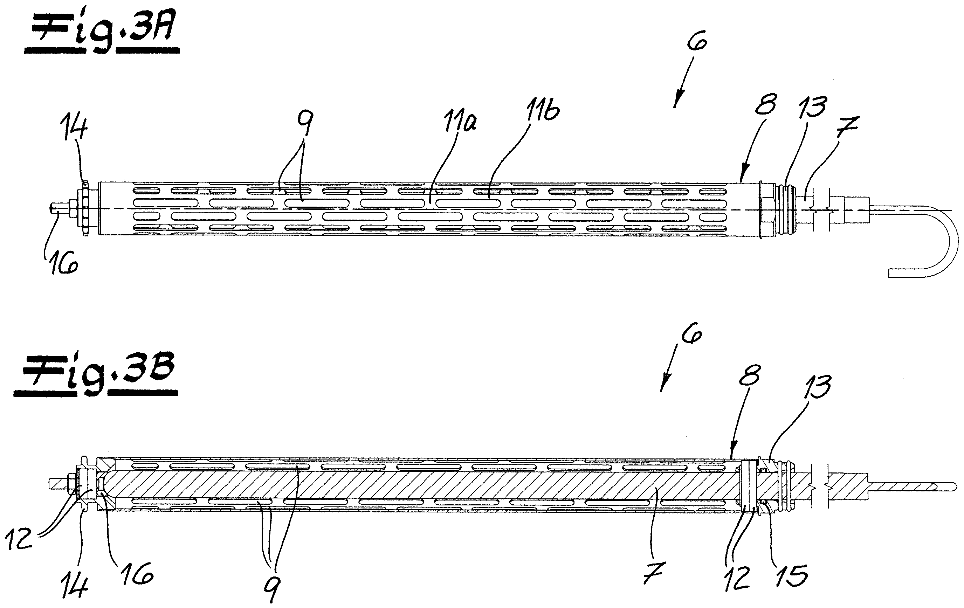

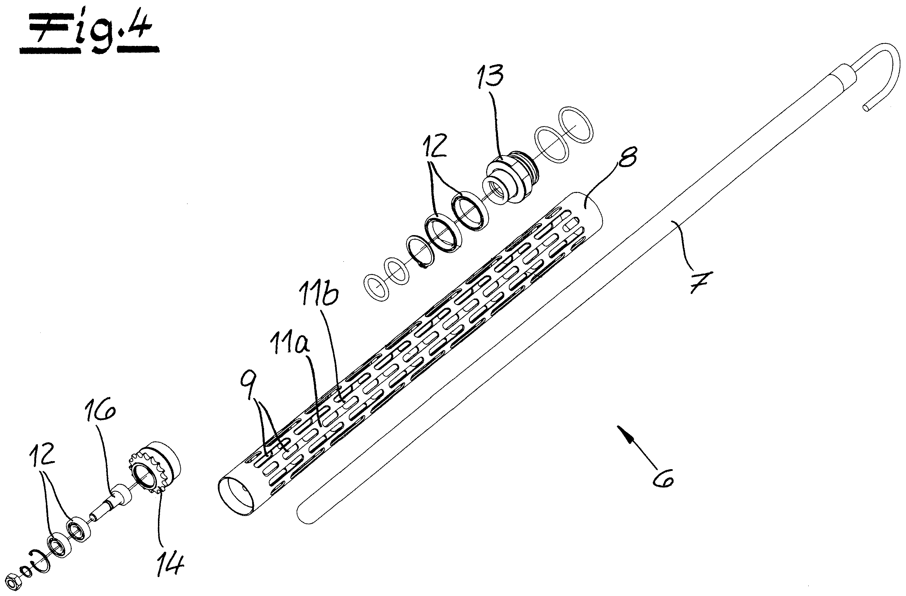

Bezüglich der mit jeweils einer ersten UV-Lichtquelle 7 ausgerüsteten Transportrollen 6, wird für die Erläuterung weiterer Details auf die Figuren 3A, 3B und 4 verwiesen. Insbesondere aus der Fig. 3A ist ersichtlich, dass die Öffnungen 9 in Form von Längsschlitzen in dem Mantel 8 vorgesehen sind, wobei zwischen den Längsschlitzen in Längsrichtung sowie in Umfangsrichtung verlaufende Stege 11a, 11b verbleiben. Die Öffnungen 9 in Form von Längsschlitzen sowie die sich in Umfangsrichtung erstreckenden Stege 11a, 11b bilden in Längsrichtung verlaufende Reihen, wobei die Öffnungen 9 der in Umfangsrichtung aufeinanderfolgenden Reihen gegeneinander versetzt sind, um eine gleichmäßige Kraftverteilung zu erreichen.

In einer vergleichenden Betrachtung der Figuren 3B und 4 ist zu erkennen, dass bei den in Rede stehenden Transportrollen 6, der Mantel 8 drehbar um die feststehende erste UV-Lichtquelle 7 angeordnet ist. Die drehbare Lagerung erfolgt dabei mit Hilfe von Wälzlagern 12, wobei die erste UV-Lichtquelle 7 durch eine Verbindungshülse 13 in den Mantel 8 eingeführt ist und an der gegenüberliegenden Seite des Mantels 8 ein Antriebsrad 14 in Form eines Zahnrades befestigt ist. Der Durchmesser des Mantels 8 beträgt beispielsweise 50 mm.

Die UV-Lichtquelle 7 ist in der Verbindungshülse 13 mittels eines O-Rings 15 fixiert, wobei das vordere Ende der UV-Lichtquelle 7 von einem Zapfen 16 begrenzt ist.

## Patentansprüche

1. Desinfektions- und Transportanordnung für den Transport und die Desinfektion von Produkten mit zumindest einer Transportrolle (6) und einer in die Transportrolle (6) eingesetzten ersten UV-Lichtquelle (7), **dadurch gekennzeichnet, dass** ein Mantel (8) der Transportrolle (6) aus einem für das von der ersten UV-Lichtquelle (7) erzeugte UV-Licht undurchlässigem Material gebildet ist und Öffnungen (9) für einen Durchtritt des UV-Lichtes aufweist.

2. Desinfektions- und Transportanordnung nach Anspruch 1, **dadurch gekennzeichnet, dass** der Mantel (8) aus Metall gebildet ist.

3. Desinfektions- und Transportanordnung nach Anspruch 1 oder 2, **dadurch gekennzeichnet, dass** der Mantel (8) Öffnungen (9) in Form von Längsschlitzen und zwischen den Längsschlitzen verlaufende Stege (11a, 11b) aufweist.

4. Desinfektions- und Transportanordnung nach einem der Ansprüche 1 bis 3, **dadurch gekennzeichnet, dass** der Flächenanteil der Öffnungen (9) an der Gesamtfläche des Mantels (8) zwischen 50 % und 80 % beträgt.

5. Desinfektions- und Transportanordnung nach einem der Ansprüche 1 bis 4, **dadurch gekennzeichnet, dass** die erste UV-Lichtquelle (7) zylinderförmig ausgestaltet und ortsfest angeordnet ist, wobei der zugeordnete Mantel (8) um die erste UV- Lichtquelle (7) drehbar gelagert ist.

6. Desinfektions- und Transportanordnung nach einem der Ansprüche 1 bis 5, **dadurch gekennzeichnet, dass** der Mantel (8) für einen drehbaren Antrieb an ein Antriebsrad (14) angeschlossen ist.

7. Desinfektions- und Transportanordnung nach einem der Ansprüche 1 bis 6, **dadurch gekennzeichnet, dass** der Mantel (8) einen Außendurchmesser zwischen 30 mm und 80 mm aufweist.

8. Desinfektions- und Transportanordnung nach einem der Ansprüche 1 bis 7, **dadurch gekennzeichnet, dass** eine Vielzahl von Transportrollen (6, 6'), welche zumindest teilweise jeweils eine erste UV-Lichtquelle (7) aufnehmen, einen Rollenförderabschnitt (2b) bildet.

9. Desinfektions- und Transportanordnung nach Anspruch 8, **dadurch gekennzeichnet, dass** die Transportrollen (6), welche eine erste UV-Lichtquelle (7) aufnehmen, in einem einen Desinfektionstunnel bildenden Gehäuse (1) angeordnet sind.

10. Desinfektions- und Transportanordnung nach Anspruch 9, **dadurch gekennzeichnet, dass** der Desinfektionstunnel einen Eingang und einen Ausgang (3) für eine durchlaufende Beschickung mit zu desinfizierenden Produkten aufweist und dass an dem Eingang sowie dem Ausgang (3) weitere Förderabschnitte anschließen.

11. Desinfektions- und Transportanordnung nach einem Ansprüche 8 bis 10, **dadurch gekennzeichnet, dass** oberhalb der Transportrollen (6, 6') zweite UV-Lichtquellen (10) angeordnet sind.

## Claims

1. A disinfecting and transport assembly for transporting and disinfecting products comprising at least one transport roller (6) and a first UV light source (7) inserted in the transport roller (6), **characterized in that** a jacket (8) of the transport roller (6) is formed from a material which is impermeable to UV light produced by the first UV light source (7) and has openings (9) for passage of the UV light.

2. The disinfecting and transport assembly according to Claim 1, **characterized in that** the jacket (8) is formed from metal.

3. The disinfecting and transport assembly according to Claim 1 or 2, **characterized in that** the jacket (8) has openings (9) in the form of longitudinal slots and webs (11a, 11b) running between the longitudinal slots.

4. The disinfecting and transport assembly according to one of Claims 1 to 3, **characterized in that** the area fraction of the openings (9) to the total area of the jacket (8) is between 50 % and 80 %.

5. The disinfecting and transport assembly according to one of Claims 1 to 4, **characterized in that** the first UV light source (7) is configured to be cylindrical and is arranged in a fixed position, wherein the associated jacket (8) is mounted rotatably about the first UV light source (7).

6. The disinfecting and transport assembly according to one of Claims 1 to 5, **characterized in that** the jacket (8) is connected to a drive wheel (14) for a rotatable drive.

7. The disinfecting and transport assembly according to one of Claims 1 to 6, **characterized in that** the jacket (8) has an external diameter between 30 mm and 80 mm.

8. The disinfecting and transport assembly according to one of Claims 1 to 7, **characterized in that** a plurality of transport rollers (6, 6'), which receive respectively one first UV light source (7), at least partially forms a roller conveyor section (2b).

9. The disinfecting and transport assembly according to Claim 8, **characterized in that** the transport rollers (6), which receive a first UV light source (7), are arranged in a housing (1) forming a disinfection tunnel.

10. The disinfecting and transport assembly according to Claim 9, **characterized in that** the disinfection tunnel has an inlet and an outlet (3) for a load running through with products to be disinfected and that further conveyor sections adjoin the inlet and the outlet (3).

11. The disinfecting and transport assembly according to one of Claims 8 to 10, **characterized in that** two UV light sources (10) are arranged above the transport rollers (6, 6').

## Revendications

1. Ensemble de désinfection et de transport, destiné à transporter et à désinfecter des produits, comprenant au moins un rouleau transporteur (6) et une première source de lumière UV (7) insérée dans le rouleau transporteur (6), **caractérisé en ce qu'**une enveloppe (8) du rouleau transporteur (6) est constituée d'une matière imperméable à la lumière UV générée par la première source de lumière UV (7) et comporte des orifices (9) pour un passage de la lumière UV.

2. Ensemble de désinfection et de transport selon la revendication 1, **caractérisé en ce que** l'enveloppe (8) est constituée de métal.

3. Ensemble de désinfection et de transport selon la revendication 1 ou 2, **caractérisé en ce que** l'enveloppe (8) comporte des orifices (9) sous la forme de fentes longitudinales et des barrettes (11a, 11b) s'écoulant entre les fentes longitudinales.

4. Ensemble de désinfection et de transport selon l'une quelconque des revendications 1 à 3, **caractérisé en ce que** le rapport de surface des orifices (9) à la surface totale de l'enveloppe (8) est compris entre 50 % et 80 %.

5. Ensemble de désinfection et de transport selon l'une quelconque des revendications 1 à 4, **caractérisé en ce que** la première source de lumière UV (7) est conçue de forme cylindrique et est placée de manière stationnaire, l'enveloppe (8) associée étant logée de manière rotative autour de la première source de lumière UV (7).

6. Ensemble de désinfection et de transport selon l'une quelconque des revendications 1 à 5, **caractérisé en ce que** pour un entraînement rotatif, l'enveloppe (8) est raccordée sur une roue d'entraînement (14).

7. Ensemble de désinfection et de transport selon l'une quelconque des revendications 1 à 6, **caractérisé en ce que** l'enveloppe (8) présente un diamètre extérieur compris entre 30 mm et 80 mm.

8. Ensemble de désinfection et de transport selon l'une quelconque des revendications 1 à 7, **caractérisé en ce qu'**une pluralité de rouleaux de transport (6, 6'), lesquels reçoivent au moins partiellement respectivement une source de lumière UV (7) forme une portion de convoyage par rouleau (2b).

9. Ensemble de désinfection et de transport selon la revendication 8, **caractérisé en ce que** les rouleaux de transport (6), lesquels reçoivent une première source de lumière UV (7) sont placés dans un carter (1) formant un tunnel de désinfection.

10. Ensemble de désinfection et de transport selon la revendication 9, **caractérisé en ce que** le tunnel de désinfection comporte une entrée et une sortie (3) pour un chargement continu avec des produits à désinfecter et **en ce que** sur l'entrée ainsi que sur la sortie (3) se raccordent des portions de convoyage supplémentaires.

11. Ensemble de désinfection et de transport selon l'une quelconque des revendications 8 à 10, **caractérisé en ce qu'**au-dessus des rouleaux de transport (6, 6') sont placées des deuxièmes sources de lumière UV (10).
